# EUROPÄISCHE PATENTANMELDUNG

(11) **EP 4 141 124 A1**
(43) Veröffentlichungstag der Anmeldung: **01.03.2023**
(21) Anmeldenummer: 21193986.3
(22) Anmeldetag: 31.08.2021
(51) Int. Cl.: C12P 7/24, C12N 9/02, C12N 9/08

(54) **VERFAHREN ZUR BIOTECHNOLOGISCHEN HERSTELLUNG VON ALDEHYDGEMISCHEN**

(71) Anmelder: Symrise AG, 37603 Holzminden Niedersachsen (DE)
(72) Erfinder: Bornscheuer, Uwe, 17489 Greifswald (DE); Kim, In Jung, 17489 Greifswald (DE); Bayer, Thomas, 8020 Graz (AT); Brack, Yannik, 17489 Greifswald (DE)
(74) Vertreter: Eisenführ Speiser

(57) **Zusammenfassung**

Die vorliegende Erfindung betrifft biotechnologische Verfahren zur Herstellung von gesättigten als auch ungesättigten Aldehyden, sowie Mischungen daraus mit mindestens einer alpha-Dioxygenase und mindestens einer Aldehyddehydrogenase. Das Verfahren kann entweder fermentativ, als Biotransformation oder enzymatisch durchgeführt werden. Weiterhin betrifft die vorliegende Erfindung ein Vektorsystem, sowie Sequenzen und rekombinante Mikroorganismen, die Enzyme umfassen / kodieren, welche zur Herstellung der erfindungsgemäßen Aldehyde und Mischungen benutzt werden können. Weiter betrifft die vorliegende Erfindung Zusammensetzungen, welche durch die Verfahren gemäß der vorliegenden Erfindung erhalten werden.

## Beschreibung

### Technisches Gebiet

Die vorliegende Erfindung betrifft biotechnologische Verfahren zur Herstellung von gesättigten und ungesättigten Aldehyden, sowie Mischungen daraus, mit Hilfe mindestens einer alpha-Dioxygenase und mindestens einer Aldehyddehydrogenase. Das Verfahren kann entweder fermentativ, als Biotransformation oder enzymatisch durchgeführt werden. Weiterhin betrifft die vorliegende Erfindung ein Vektorsystem, sowie Sequenzen und rekombinante Mikroorganismen, die Enzyme umfassen / kodieren, welche zur Herstellung der erfindungsgemäßen Aldehyde und Mischungen benutzt werden können. Weiter betrifft die vorliegende Erfindung Zusammensetzungen, welche durch die Verfahren gemäß der vorliegenden Erfindung erhalten werden.

### Hintergrund der Erfindung

Auf dem Gebiet der industriellen Gewinnung von Geschmacksstoffen besteht ein ständiger Bedarf an effizienten und kostengünstigen Wegen der Synthese von Geschmacksstoffen. Ein Beispiel für derartige Geschmacksstoffe sind (un)gesättigte Aldehyde. Ein beispielhafter Aldehyd ist 8Z-Tetradecenal oder 1-Decenal. Diese Stoffklasse kommt vor allem in Fleischzubereitungen und Zitrusölen vor. Derzeit ist jedoch noch kein allgemein anwendbares biotechnologisches Verfahren zur Herstellung dieser Aldehyde und der korrespondierenden Säuren beschrieben worden.

Aus dem Stand der Technik sind Prozesse zur Herstellung von ungesättigten Aldehyden bekannt. So lässt sich zum Beispiel 8Z,11Z-Heptadecadienal durch die chemische Oxidation des ungesättigten Alkohols erhalten (JP 63233914). Verschiedene Aldehyde lassen sich zudem in bekannter Weise durch alpha-Oxidation mittels pflanzlicher Enzyme aus Fettsäuren herstellen, wie in WO 2012/025629 A1 beschrieben. Die Nutzung von Algenbiomasse zur Herstellung ungesättigter Aldehyde ist ebenfalls bekannt *("Concise synthesis of (8Z,11Z,14Z)-8,11,14-heptadecatrienal, (7Z,10Z,3Z)-7,10,13-hexadecatrienal, and (8Z,11Z)-8,11-heptadecadienal, components of the essential oil of marine green alga Ulva pertusa"*, Biosci Biotechnol Biochem. 2005 Jul;69(7):1348-52). Weiterhin sind Prozesse zur Herstellung von 4,7,10,13,16-Nonadecapentaenal oder 3,6,9,12,15,18-Heneicosahexaenal bekannt (*Preparation of halopolyenes and their intermediates,* JP05000974A*).*

Weiterhin sind Verfahren, die ganz allgemein Aldehyddehydrogenasen verwenden, bekannt, um Aldehyde zu Säuren zu oxidieren (Takeru Ishigeet al., Appl. Environ. Microbiol. 2000, 66, 3481-3486; Tomohisa Kato et al., Extremophiles 2010, 14, 33.).

Die voran genannten chemischen Verfahren sowie Verfahren ausschließlich basierend auf der Verwendung eines sehr speziellen Enzyms alleine besitzen jedoch den Nachteil, dass hierdurch kein umfassendes Spektrum an Aldehyden bzw. von Gemischen davon zugänglich ist. Weiterhin beruhen manche der Verfahren auf der Umsetzung mit chemischen Reagenzien sowie Katalysatoren, weshalb solche Verfahren nicht als natürliche Prozesse im Sinne der EG 1334/2008 gelten.

Ein kombiniertes Verfahren, welches eine spezifische alpha-Dioxygenase mit einer Aldehyddehydrogenase kombiniert und das Produktionsspektrum gezielt erweitert, ist nicht bekannt. Bei einem rekombinanten Reaktionssystem mit zwei oder mehr Enzymen, wobei die gewünschten katalytischen Reaktionen sequenziell und hinsichtlich der Substratspezifität und -selektivität spezifisch ablaufen sollen, ist es nötig die verwendeten Enzyme genau aufeinander abzustimmen, sodass diese nicht durch, z.B. im Fall einer Ganzzell-Katalyse, endogene Enzyme des Produktionsorganismus beeinträchtigt werden. Die Reaktionsführung ist daher entscheidend, damit die Zwischenprodukte auch vom zweiten oder jeweils nächsten Enzym in der Kaskade erkannt werden und für dieses zur Verfügung stehen. Eine solche Abstimmung bzw. Kombination ist technisch stets anspruchsvoll und nicht geradlinig prognostizierbar, sondern bedarf vielmehr intensiver Analysen zu den Enzymen von Interesse im jeweiligen biotechnologischen Kontext.

Das natürliche Vorkommen einiger ungesättigter Aldehyde mit ein oder mehreren Doppelbindungen wie z.B. 8Z-Heptadecenal, 8Z,11Z-Heptadecadienal oder 8*Z*,11*Z*,14*Z-*Heptadecatrienal wurde in Presssäften von Gurke (*Cucumber Aroma formation of cucumber (Cucumis sativus L.) and bitter gourd (Momordica charantia L.) by saltsqueezing,* Journal of Home Economics of Japan Vol. 60 (2009) No. 10 p. 877-885) oder der Alge *Ulva pertusa* beschrieben (*Production of bioflavor by regeneration from protoplasts of Ulvapertusa (Ulvales, Chlorophyta)* Hydrobiologia, 1990, Vol. 204, pp 143-149). Der Gehalt der Aldehyde 8Z-Heptadecenal, 8*Z*,11*Z*-Heptadecadienal, 8Z,11Z,14Z-Heptadecatrienal wird in der Literatur mit 2%, 19,9% und 26,1% angegeben.

Der Geruch von 8Z,11Z-Heptadecadienal wird als typisch für Algen beschrieben *(Production of bioflavor by regeneration from protoplasts of Ulva pertusa (Ulvales, Chlorophyta),* Hydrobiologia, 1990, Vol. 204, pp 143-149*).*

Der Geschmack von 4Z,7Z,10Z,13Z-Nonadecatetraenal, 4Z,7Z,10Z,13Z,16Z-Nonadecapentaenal oder 3,6,9,12,15,18-Heneicosahexaensäure ist dagegen nicht beschrieben.

8Z-Pentadecenal ist als natürlich vorkommender Bestandteil von Gurken bekannt (*Kemp,* A *C15 aldehyde from Cucumis sativus,* Phytochemistry, Volume 16, Issue 11, 1977, Pages 1831-1832*).* Der Geschmack von 8Z-Pentadecenal wird vor allem als ölig beschrieben und auch die geschmacksverbessernden Eigenschaften auf Hühnchen und gegrilltes Rindfleisch sind beschrieben. Weiterhin ist ein Verfahren zur Herstellung aus 1-Nonin und 6-Brom-1-hexanol bekannt, was jedoch nicht als natürliches Herstellverfahren nach EC 1334/2008 klassifiziert werden kann (JP 2014043409).

Der Geschmack von 4Z,7Z,10Z,13Z-Nonadecatetraenal ist nicht bekannt und es existierten bislang auch keine Herstellverfahren.

Verschiedene Aldehyde lassen sich durch alpha-Oxidation mittels pflanzlicher Enzyme aus Fettsäuren herstellen, wie in WO 2012/025629 A1 beschrieben. Die Nutzung von Algenbiomasse zur Herstellung ungesättigter Aldehyde ist ebenfalls bekannt.

Aus dem Stand der Technik sind zudem weitere Verfahren zur Herstellung von Fettsäuren aus den entsprechenden Ölen oder Fetten durch enzymatische Spaltung bekannt. Hierzu werden vor allem bakterielle Lipasen wie zum Beispiel Enzyme aus *Aspergillus niger*, *Rhizopus oryzae, Penicillium camembertii, Mucor juvanicus, Penicillium roquefortii,* Schweinepankreas, *Candida rugosa*, *Rhizomucor miehei*, *Candida antarctica* oder *Rhizops* *delemar* verwendet (*Industrial applications of microbial lipases*, Enzyme and Microbial Technology Volume 39, Issue 2, 26 June 2006, Pages 235-251).

Aufgabe der vorliegenden Erfindung war es daher, ein Verfahren zur Herstellung von Aldehyden oder Aldehydgemischen bereitzustellen, welches als natürliches Herstellverfahren nach EC 1334/2008 klassifiziert werden kann. Ferner war es eine Aufgabe, durch die gezielte Abstimmung von enzymatischen Stoffwechselschritten ein integriertes biotechnologisches Verfahren bereitzustellen, das ohne chemische Zwischenschritte die Produkte von Interesse in hoher Ausbeute und Reinheit bereitstellen kann.

### Zusammenfassung der Erfindung

Die vorliegende Erfindung betrifft demnach gemäß einem primären Aspekt das Bereitstellen eines Verfahrens zur Herstellung von gesättigten und ungesättigten Aldehyden mit Hilfe mindestens einer alpha-Dioxygenase mit einer Aminosäuresequenz gemäß SEQ ID NO: 1 oder 2 oder einer Aminosäuresequenz mit einer Ähnlichkeit von 90 %, 91 %, 92 %, 93 %, 94 %, 95 %, 96 %, 97%, 98 %, 99% oder mehr zu SEQ ID NO: 1 oder 2 und mindestens einer Aldehyddehydrogenase.

Weiterhin betrifft die vorliegende Erfindung gemäß einer Ausführungsform ein fermentatives Verfahren zur Herstellung von gesättigten und ungesättigten Aldehyden und Mischungen daraus. In einer anderen Ausführungsform des erfindungsgemäßen Verfahrens wird ein enzymatisches Verfahren bereitgestellt.

In einer weiteren Ausführungsform betrifft das vorliegende erfindungsgemäße Verfahren die Nutzung von Edukten, welche entweder biotechnologischen, natürlichen oder chemischen Ursprungs sind.

In wiederum einer weiteren Ausführungsform betrifft das erfindungsgemäße Verfahren bevorzugt eingesetzte Edukte ausgewählt aus der Gruppe der Carbonsäuren und mit Alkoholen veresterten Carbonsäuren.

Eine weitere Ausführungsform betrifft das erfindungsgemäße Verfahren, wobei eine Carbonsäure als ein erstes Edukt erhalten wird, welche als sodann als modifiziertes Edukt für ein biotechnologisches Verfahren zur Herstellung von gesättigten und ungesättigten Aldehyden mit Hilfe mindestens einer alpha-Dioxygenase und mindestens einer Aldehyddehydrogenase eingesetzt wird.

Weiterhin betrifft das erfindungsgemäße Verfahren bevorzugte Produktmischungen, Mikroorganismen, die im Verfahren eingesetzt werden sowie Aminosäuresequenzen der bevorzugt einzusetzenden Enzyme und Nukleotidsequenzen bzw. Nukleinsäure-Abschnitte kodierend für die einzusetzenden Enzyme.

Ein weiterer Aspekt der vorliegenden Erfindung betrifft die Bereitstellung eines Vektorsystems, welches die erfindungsgemäß verwendeten Enzyme kodiert.

### Kurze Beschreibung der Abbildungen

**Figur 1** zeigt das Ergebnis der Substratspezifitätsversuche in relativer Aktivität bei verschiedenen Fettsäuresubstraten.
**Figur 2** zeigt das Ergebnis der Ganzzellbiotransformation von Caprinsäure und Myristinsäure. **Figur 2A** zeigt die Ergebnisse mit einer alpha-Dioxygenase gemäß SEQ ID NO: 2 und **Figur 2B** zeigt die Ergebnisse mit einer alpha-Dioxygenase gemäß SEQ ID NO: 1.

### Kurze Beschreibung der Sequenzen

SEQ ID NO: 1: Aminosäuresequenz, die für eine Alpha-Dioxygenase aus *Leptolyngbya sp.* PCC 7375 kodiert.
SEQ ID NO: 2: Aminosäuresequenz, die für eine Alpha-Dioxygenase aus *Calothrix* sp. PCC 6303kodiert.
SEQ ID NO: 3: Nukleinsäuresequenz, die für eine Alpha-Dioxygenase aus *Leptolyngbya sp.* PCC 7375 kodiert.
SEQ ID NO: 4: Nukleinsäuresequenz, die für eine Alpha-Dioxygenase aus *Calothrix* sp. PCC 6303 kodiert.
SEQ ID NO: 5: Aminosäuresequenz, die für eine NAD(P)H-Oxidase aus *Lactobacillus sanfranciscensis* kodiert.
SEQ ID NO: 6: Aminosäuresequenz, die für eine NADH-Oxidase aus *Lactobacillus brevis* kodiert.
SEQ ID NO: 7: Nukleinsäuresequenz, die für eine NAD(P)H-Oxidase aus *Lactobacillus sanfranciscensis* kodiert.
SEQ ID NO: 8: Nukleinsäuresequenz, die für eine NADH-Oxidase aus *Lactobacillus brevis* kodiert.
SEQ ID NO: 9: Aminosäuresequenz, die für das Enzym VhFALDH aus *Vibrio harveyi* kodiert.
SEQ ID NO: 10: Aminosäuresequenz, die für die 175Q Mutante des Enzyms VhFALDH aus *Vibrio harveyi* kodiert.
SEQ ID NO: 11: Aminosäuresequenz, die für das Enzym ReALDH aus *Rhodococcus erythropolis* UPV-1 kodiert.
SEQ ID NO: 12: Aminosäuresequenz, die für das Enzym GtALDH aus *Geobacillus thermodenitrificans* kodiert.
SEQ ID NO: 13: Aminosäuresequenz, die für das Enzym Maqu3410, eine Aldehyddehydrogenase aus *Marinobacter aquaeolei* VT8 kodiert.
SEQ ID NO: 14: Aminosäuresequenz, die für das Enzym AId1 aus *Acinetobacter* sp.10 Stamm M1 kodiert.
SEQ ID NO: 15: Aminosäuresequenz, die für das Enzym HFD4, eine Aldehyddehydrogenase aus *Yarrowia lipolytica* kodiert.

### Definitionen

Der Begriff Vektorsystem wie hierein verwendet, bezeichnet ein System, das aus mindestens einem oder mehreren Vektoren oder Plasmidvektoren besteht oder diese enthält. So kann ein Vektorsystem einen (Plasmid)vektor enthalten, welcher mehrere unterschiedliche Zielgene kodiert. Ein Vektorsystem kann darüber hinaus auch mehrere (Plasmid)vektoren enthalten, die ihrerseits mindestens ein Zielgen gemäß der vorliegenden Offenbarung enthalten. Ein Vektorsystem kann somit nur ein (Plasmid)vektorkonstrukt oder mehrere (Plasmid)vektorkonstrukte umfassen, wobei letztere gleichzeitig oder hintereinander in den entsprechenden rekombinanten Wirtsorganismus stabil oder transient transformiert werden können, sodass die von den einzelnen Konstrukten kodierten Zielgene von dem Wirtsorganismus transkribiert und translatiert werden können.

Die Begriffe Aminosäure, Protein und Polypeptid werden hierin austauschbar verwendet. Die hierein offenbarten Aminosäuren, oder funktionale Abschnitte davon, haben nach korrekter Faltung enzymatische Funktion. Demnach wird auch der Begriff Enzym hierin austauschbar für den Begriff Aminosäure verwendet.

Wann immer die vorliegende Offenbarung auf Sequenzhomologien oder Sequenzidentitäten von Nuklein- oder Proteinsequenzen in Form von Prozentangaben Bezug nimmt, beziehen sich diese Angaben auf Werte, wie sie unter Verwendung von EMBOSS Water Pairwise Sequence Alignments (Nucleotide) für Nukleinsäuresequenzen bzw. EMBOSS Water Pairwise Sequence Alignments (Protein) für Aminosäuresequenzen berechnet werden können. Bei vom European Molecular Biology Laboratory (EMBL) European Bioninformatics Institute (EBI) zur Verfügung gestellten Tools für lokale Sequenzalignments verwenden einen modifizierten Smith-Waterman Algorithmus. Ferner wird hierbei bei der Durchführung des jeweiligen paarweisen Alignments zweier Sequenzen unter Verwendung des modifizierten Smith-Waterman Algorithmus auf die vom EMBL-EBI derzeit angegebenen Default Parameter Bezug genommen. Diese lauten (i) für Aminosäuresequenzen: Matrix = BLOSUM62, Gap open penalty = 10 und Gap extend penalty = 0,5 sowie (ii) für Nukleinsäuresequenzen: Matrix = DNAfull, Gap open penalty = 10 und Gap extend penalty = 0,5. Die Berechnung einer Sequenzhomologie oder Sequenzidentität ist immer über die Gesamtlänge einer Sequenz zu berechnen.

Die Anzucht und Kultivierung, Isolierung sowie Aufreinigung eines rekombinanten Mikroorganismus oder Pilzes bzw. eines Proteins bzw. Enzyms, welches von einer Nukleinsäure gemäß der Offenbarung der vorliegenden Erfindung kodiert wird, ist dem Fachmann auf dem Gebiet bekannt.

Die Begriffe Protein, Polypeptid und Enzym werden auf Grund der stets enzymatischen Funktion der hierin offenbarten Genprodukte austauschbar verwendet. Ebenso werden die Begriffe Gen und Nukleinsäure(abschnitt) für die Zwecke der vorliegenden Offenbarung austauschbar verwendet.

Die Nukleinsäuren, Nukleotidsequenzen oder Nukleinsäure-Abschnitte (diese Begriffe werden austauschbar verwendet für DNS Sequenzen, welche ein funktionales Enzym, oder einen funktionalen Bereich davon, kodieren), welche gemäß der vorliegenden Erfindung zur Expression eines gewünschten Zielproteins verwendet werden, können gegebenenfalls Codon-optimiert sein, d.h. die Codon-Verwendung eines Genes wird auf die des als Expressionsstamm gewählten rekombinanten Mikroorganismus oder Pilzes angepasst. Es ist dem Fachmann auf dem Gebiet bekannt, dass ein gewünschtes Zielgen, welches ein Protein von Interesse kodiert, ohne Änderung der translatierten Proteinsequenz modifiziert werden kann, um der spezifischen Spezies-abhängigen Codon-Verwendung Rechnung zu tragen. So können die zu transformierenden Nukleinsäuren der vorliegenden Erfindung spezifisch auf die Codon-Verwendung von E. *coli* oder eines anderen Bakteriums, von *Saccharomyces* spp. oder einer anderen Hefe angepasst werden.

Die offenbarten Carbonsäuren können entweder in ihrer freien Form oder verestert an Alkohole, welche etwa als Bestandteile von Lipiden vorkommen, vorliegen.

Sofern die Konfiguration(en) von (Z)/(E) Isomeren hierein für eine genannte Verbindung nicht speziell angegeben ist, so umfasst die Angabe alle möglichen Konfigurationsisomere der entsprechenden Verbindung, oder eine Mischung davon. Es gilt anzumerken, dass gewisse hierin genannte Enzyme dazu in der Lage sind, beide Isomere einer Verbindung von Interesse zu erkennen und umzusetzen.

### Ausführliche Beschreibung

Die Aufgabe der vorliegenden Erfindung wird primär durch Bereitstellen eines biotechnologischen Verfahrens zur Herstellung mindestens eines ungesättigten Aldehyds und dessen korrespondierender Carbonsäure und/oder mindestens eines gesättigten Aldehyds und dessen korrespondierender Carbonsäure gelöst, wobei das Verfahren das Bereitstellen mindestens einer alpha-Dioxygenase mit einer Aminosäuresequenz gemäß SEQ ID NO: 1 oder 2, oder einer Aminosäuresequenz mit einer Ähnlichkeit von 90 %, 91 %, 92 %, 93 %, 94 %, 95 %, 96 %, 97%, 98 %, 99% oder mehr zu SEQ ID NO: 1 oder 2 und mindestens einer Aldehyddehydrogenase umfasst.

Der erhaltene Aldehyd ist dabei immer um ein Kohlenstoffatom verkürzt im Vergleich zu dem eingesetzten Edukt. Ein gesättigter Aldehyd bezieht sich im Kontext der vorliegenden Erfindung auf einen Aldehyd ohne Doppelbindungen, wobei ein ungesättigter Aldehyd sich auf Aldehyde mit vorhandenen Doppelbindungen bezieht. Die korrespondierende Carbonsäure ist hierbei eine Verbindung mit gleicher Kettenlänge und gleichem Sättigungsgrad wie der erhaltene Aldehyd, welche aber mindestens eine Carboxylgruppe trägt. Die erfindungsgemäß verwendete alpha-Dioxygenase gemäß SEQ ID NO: 1 oder 2, oder einer Aminosäuresequenz mit einer Ähnlichkeit von 90 %, 91 %, 92 %, 93 %, 94 %, 95 %, 96 %, 97%, 98 %, 99% oder mehr zu SEQ ID NO: 1 oder 2, katalysiert die Oxidation von Carbonsäuren der Kettenlänge C6 - C22 am alpha-Kohlenstoffatom, sodass der zur Carbonsäure korrespondierende und um ein Kohlenstoffatom verkürzte Aldehyd entstehen kann.

Die erfindungsgemäß zu verwendenden alpha-Dioxygenase gemäß SEQ ID NO: 1 oder 2 besitzen den Vorteil, dass diese ein besonders vorteilhaftes Substrat- und Produktspektrum besitzen. Die erfindungsgemäßen alpha-Dioxygenase aus *Leptolyngbya* sp. (SEQ ID NO: 1) und *Calothrix parientina* (SEQ ID NO: 2) weisen eine bevorzugte Aktivität gegenüber kurzen Fettsäuren mit 10 bis 14 Kohlenstoffatomen auf. Gleichzeitig ist das Substratspektrum nicht sehr eng, so dass sich diese Enzymvarianten besonders vorteilhaft in den hierin offenbarten Verfahren einsetzen lassen.

Die mindestens eine verwendete Aldehyddehydrogenase katalysiert aufgrund ihrer Substratspezifität die Oxidation eines Aldehyds zur korrespondierenden Carbonsäure, die wiederum weiter umgesetzt werden kann zu einem um ein Kohlenstoffatom verkürzten Aldehyd mit der alpha-Dioxygenase. Somit kann man mit dem erfindungsgemäßen Verfahren gezielt Aldehydmischungen mit Aldehyden herstellen, die unterschiedliche Kettenlängen besitzen.

In einer Ausführungsform des erfindungsgemäßen Verfahrens kann die alpha-Oxidation und die Aldehyd-Oxidation gleichzeitig erfolgen, wobei in einer weiteren Ausführungsform erst die alpha-Oxidation und dann die Aldehyd-Oxidation durchgeführt werden kann, wobei der Reaktionszyklus in beiden Ausführungsformen bis zum Erhalt des gewünschten Produktes fortgesetzt werden kann.

Besonders bevorzugt im Kontext der vorliegenden Erfindung ist es, wenn die durch alpha-Oxidation erhaltenen Fettsäuren als bevorzugte Substrate für die Aldehyd-Oxidation verwendet werden.

In einer bevorzugten Ausführungsform des erfindungsgemäßen Verfahrens kann das biotechnologische Herstellungsverfahren ein fermentatives Verfahren sein, welches die folgenden Schritte umfasst:
i. Bereitstellen mindestens eines rekombinanten Mikroorganismus oder Pilzes enthaltend einen Nukleinsäure-Abschnitt, umfassend mindestens ein für eine alpha-Dioxygenase kodierendes Gen und/oder mindestens ein für eine Aldehyddehydrogenase kodierendes Gen mit einer Nukleinsäuresequenz gemäß einer der SEQ ID NO: 3 oder 4 oder einer Nukleinsäuresequenz mit einer Ähnlichkeit von 90 %, 91 %, 92 %, 93 %, 94 %, 95 %, 96 %, 97%, 98 %, 99% oder mehr zu einer der SEQ ID NO: 3 oder 4;
ii. Kultivieren des mindestens einen rekombinanten Mikroorganismus unter Bedingungen, die die Expression des entsprechenden Expressionsproduktes erlauben;
iii. Hinzufügen mindestens einer Carbonsäure oder mindestens einer mit einem Alkohol veresterten Carbonsäure zu dem mindestens einen kultivierten, rekombinanten Mikroorganismus;
iv. Erhalten mindestens eines ungesättigten Aldehyds und dessen korrespondierender Carbonsäure und/oder mindestens eines gesättigten Aldehyds und dessen korrespondierender Carbonsäure durch Reaktion der mindestens einen alpha-Dioxygenase und der mindestens einen Aldehyddehydrogenase mit der mindestens einen Carbonsäure oder der an einen Alkohol veresterten Carbonsäure aus Schritt iii.

Ein fermentatives Verfahren im Kontext der vorliegenden Erfindung bezieht sich auf ein Verfahren, bei dem in mindestens einem Schritt ein rekombinanter Mikroorganismus kultiviert wird, welcher die mindestens eine alpha-Dioxygenase und die mindestens eine Aldehyddehydrogenase exprimiert, die zur Herstellung des erfindungsgemäßen Produktes erforderlich sind. Das Expressionsprodukt ist hierbei die mindestens eine alpha-Dioxygenase gemäß SEQ ID NO: 1 oder 2 oder einer Aminosäuresequenz mit einer Ähnlichkeit von 90 %, 91 %, 92 %, 93 %, 94 %, 95 %, 96 %, 97%, 98 %, 99% oder mehr zu einer der SEQ ID NO: 1 oder 2 und/oder die mindestens eine Aldehyddehydrogenase. Dabei kann die Umsetzung des Edukts gemäß einer Ausführungsform durch die Sekretion des Enzyms in den Reaktionsansatz, in welchem die Edukte vorliegen, stattfinden, wohingegen gemäß einer weiteren Ausführungsform die Umsetzung im Zytosol des mindestens einen Mikroorganismus stattfinden kann und das Produkt anschließend sekretiert werden kann.

In wiederum einer weiteren bevorzugten Ausführungsform kann das fermentative Verfahren eine Biotransformation mit ruhenden Zellen und/oder Mikroorganismen sein, umfassend einen zusätzlichen Schritt (ii.a), umfassend das Entfernen des Kultivierungsmediums und Aufnahme des kultivierten, rekombinanten Mikroorganismus in einen wässrigen Puffer. Die in den Zellen vorhandenen Expressionsprodukte katalysieren die Umsetzung der in Schritt iii. hinzugefügten mindestens einen Carbonsäure oder mindestens einen mit einem Alkohol veresterten Carbonsäure, um in Schritt iv. mindestens ein ungesättigtes Aldehyd und dessen korrespondierende Carbonsäure und/oder mindestens ein gesättigtes Aldehyds und dessen korrespondierender Carbonsäure zu erhalten.

Bereitstellen im Kontext der vorliegenden Erfindung bedeutet immer das Involvieren eines aktiven Schrittes zur Verfügbarmachung eines Edukts, eines Enzyms oder ähnlichem. Gemäß einer Ausführungsform kann das Bereitstellen immer einen technischen Schritt, wie zum Beispiel die Klonierung eines Reaktionsorganismus oder die Herstellung eines Edukts sein.

In einer weiteren bevorzugten Ausführungsform des erfindungsgemäßen Verfahrens kann das biotechnologische Herstellungsverfahren ein enzymatisches Verfahren sein, welches die folgenden Schritte umfasst:
i. Bereitstellen mindestens einer alpha-Dioxygenase mit einer Aminosäuresequenz gemäß SEQ ID NO: 1 oder 2 oder einer Aminosäuresequenz mit einer Ähnlichkeit von 90 %, 91 %, 92 %, 93 %, 94 %, 95 %, 96 %, 97%, 98 %, 99% oder mehr zu einer der SEQ ID NO: 1 oder 2 und mindestens einer Aldehyddehydrogenase, wobei die mindestens eine alpha-Dioxygenase und/oder die mindestens eine Aldehyddehydrogenase natürlich, chemisch oder biotechnologisch hergestellt sein kann/können;
ii. Hinzufügen mindestens einer Carbonsäure oder mindestens einer mit einem Alkohol veresterten Carbonsäure zu den bereitgestellten Enzymen aus Schritt i;
iii. Erhalten mindestens eines ungesättigten Aldehyds und dessen korrespondierender Carbonsäure und/oder mindestens eines gesättigten Aldehyds und dessen korrespondierender Carbonsäure durch Reaktion der mindestens einen alpha-Dioxygenase und der mindestens einen Aldehyddehydrogenase mit der mindestens einen Carbonsäure oder der an einen Alkohol veresterten Carbonsäure aus Schritt ii.

Ein enzymatisches Verfahren im Kontext der vorliegenden Erfindung bezeichnet ein Verfahren, bei dem die mindestens eine alpha-Dioxygenase und die mindestens eine Aldehyddehydrogenase gereinigt oder partiell gereinigt außerhalb eines Mikroorganismus vorliegen können. In einer Ausführungsform können die beiden Enzyme als Zelllysat vorliegen, welches den Zustand von nicht mehr kultivierbaren Mikroorganismen bezeichnet, die durch physikalische, chemische oder enzymatische Prozesse aufgeschlossen wurden. In einer weiteren Ausführungsform können die Enzyme mit einer Reinheit von > 80% vorliegen, wobei diese durch ein dem Fachmann geläufigen Reinigungsverfahren gereinigt wurden. In wiederum einer weiteren Ausführungsform können die Enzyme mit einer Reinheit < 80% vorliegen, wobei diese durch ein dem Fachmann geläufigen Reinigungsverfahren partiell gereinigt wurden. In noch einer weiteren Ausführungsform können die Enzyme durch Proteinsynthese gewonnen und optional aufgereinigt werden.

Ein Edukt biotechnologischen Ursprungs ist ein durch Fermentation von Mikroorganismen hergestelltes Edukt, wobei ein Edukt natürlichen Ursprungs aus einer natürlichen Quelle, zum Beispiel einer Pflanze, einem Pilz, einem Tier, einem Mikroorganismus oder aus dem Boden gewonnen werden kann. Ein Edukt chemischen Ursprungs kann durch ein chemisches Syntheseverfahren aus Vorstufen des Edukts hergestellt werden.

In einer weiteren bevorzugten Ausführungsform kann mindestens eines der Edukte des erfindungsgemäßen Verfahrens biotechnologischen, natürlichen oder chemischen Ursprungs sein. Für die Edukte biotechnologischen, natürlichen oder chemischen Ursprung gilt das voran Gesagte.

Gemäß einer weiteren bevorzugten Ausführungsform des erfindungsgemäßen Verfahrens können die Edukte ausgewählt sein aus der Gruppe bestehend aus vollständig gesättigten, ein-, zwei- oder dreifach ungesättigten oder einfach verzweigten Fettsäuren von C6 bis C20, bevorzugt von C8 bis C18, besonders bevorzugt aus der Gruppe bestehend aus Caprylsäure, Caprinsäure, 4-Ethyloctansäure, Laurinsäure, 10-Methylundecansäure, 9-Methylundecansäure, 10-Methyldodecansäure, 11-Methyldodecansäure, 11-Methyltridecansäure, 12-Methyltridecansäure, 13-Methyltetradecansäure, 12-Methyltetradecansäure, 13-Methylpentadecansäure, 14-Methylpentadecansäure, 14-Methylhexadecansäure, 15-Methylhexadecansäure, Myristinsäure, Palmitinsäure, Stearinsäure, Palmitoleinsäure, Petroselinsäure, Margarinsäure, cis-Vaccensäure, Linolsäure, Stearidonsäure, gamma-Linolensäure, alpha-Linolensäure, Ölsäure, Punicinsäure, alpha-Elaeostearinsäure sowie der zugrundeliegenden fetten Fettsäureester, insbesondere die fetten Öle ausgewählt aus Olivenöl, Erucasäure-armem Rapsöl, Macadamianussöl, Sanddornfruchtfleischöl, Borretschöl, Johannisbeerkernöl, Petersiliensamenöl, Dillsamenöl, Granatapfelkernöl, Kokosnussöl, Sonnenblumenöl, Weizenkeimöl, Reiskeimöl, Erdnussöl, Sesamöl, Palmfruchtöl, Traubenkernöl sowie Pilzöle, gewonnen aus einer beliebigen der Gattungen, ausgewählt aus *Conidiobolus, Flammulina, Fomes, Ganoderma, Mortierella, Panellus, Pleurotus, Psathyrella, Stereum, Umbelopsis* und deren Derivate sind.der Carbonsäuren und der mit Alkoholen veresterten Carbonsäuren, bevorzugt, wobei die Edukte ausgewählt sind aus der Gruppe bestehend aus Palmitoleinsäure, Arachidonsäure, alpha-Linolensäure, Ölsäure, Punicinsäure, alpha-Elaeosterarinsäure, Docosahexaensäure, Eicosapentaensäure, Petroselinsäure, Chaulmoograsäure, alpha-Licarsäure, Olivenöl, Rapsöl, Macadamianussöl, Sanddornfruchtfleischöl, Tungöl, Fischöl, Borretschöl, Chaulmoograöl, Petersilienöl, Oiticicaöl, Granatapfelkernöl, Kokosnussöl, Sonnenblumenöl, Traubenkernöl sowie Pilzöle, gewonnen aus einer beliebigen der Gattungen, ausgewählt aus *Conidiobolus, Flammulina, Fomes, Ganoderma, Mortierella, Panellus, Pleurotus, Psathyrella, Stereum, Umbelopsis* und deren Derivaten.

In einer weiteren bevorzugten Ausführungsform kann das Verfahren die weiteren Schritte:
(a) Erhalten mindestens einer Carbonsäure, bevorzugt mindestens einer Carbonsäure wie hierin definiert, bevorzugt umfassend eine ungerade Anzahl an Kohlenstoffatomen, als Produkt;
(b) optional: Reinigen der mindestens einen als Produkt erhaltenen Carbonsäure;
(c) Verwenden der mindestens einen als Produkt erhaltenen Carbonsäure als modifiziertes Edukt zur Durchführung eines biotechnologischen Verfahrens zur Herstellung mindestens eines ungesättigten Aldehyds und dessen korrespondierender Carbonsäure und/oder mindestens eines gesättigten Aldehyds und dessen korrespondierender Carbonsäure, wobei das Verfahren das Bereitstellen mindestens einer alpha-Dioxygenase und mindestens einer Aldehyddehydrogenase umfasst, bevorzugt wobei das Verfahren ein Verfahren gemäß der vorliegenden Erfindung ist;
umfassen.

Ein "modifiziertes Edukt" im Rahmen der vorliegenden Erfindung, ist ein Edukt welches nicht dasselbe Edukt wie die im erfindungsgemäßen Verfahren einzusetzende Fett- oder Carbonsäure ist. Dieses modifizierte Edukt kann das Produkt einer zuvor durchgeführten enzymatischen Umsetzung mit einer alpha-Dioxygenase und einer Aldehyddehydrogenase wie hierin beschrieben sein.

In einer bevorzugten Ausführungsform kann das Verfahren gemäß der vorliegenden Erfindung als Zyklus-Reaktion durchgeführt werden. Dies bedeutet, dass Carbonsäuren, bevorzugt Fettsäuren, mit einer ungeraden Anzahl an Kohlenstoffatomen, wie diese durch ein oben beschriebenes Verfahren als Produkt erhalten werden können, sogleich in einem integrierten Prozess erneut als Edukt verwendet werden, wobei mindestens eine der Fettsäuren des erfindungsgemäßen Verfahrens biotechnologischen, natürlichen oder chemischen Ursprungs ist. Für die Edukte biotechnologischen, natürlichen oder chemischen Ursprung gilt das voran Gesagte.

In einer weiteren bevorzugten Ausführungsform kann das Produkt des erfindungsgemäßen Verfahrens mindestens ein gesättigter Aldehyd und dessen korrespondierende Carbonsäure und/oder ein ungesättigter Aldehyd und dessen korrespondierende Carbonsäure sein. Hierbei können die Produkte des erfindungsgemäßen Verfahren vorzugsweise ausgewählt aus der Gruppe umfassend 7Z,10Z-Hexadecadienal, 8Z,11Z-Heptadecadienal, 6*E*,9*E*-Pentadecadienal, 7Z-Hexadecenal, 8Z-Pentadecenal, 7Z-Tetradecenal, 8Z,11Z,14Z-Heptadecatrienal, 7*Z*,10*Z*,13*Z*-Hexadecatrienal, 4Z,7Z,10Z,13Z-Nonadecatetraenal, 8*Z*,10*E*,12*E*-Heptadecatrienal, 8*Z*,10*E*,12*Z-*Heptadecatrienal, 7Z,9E,11Z-Hexadecatrienal, 7Z,9E,11E-Hexadecatrienal, 9-Decenal, 10-Undecenal, 3Z-Decenal, 4Z-Undecenal, 7Z-Dodecenal, 8Z-Tridecenal, 7Z-Tetradecenal, 8Z-Pentadecenal, 9Z-Tetradecenal, 10Z-Pentadecenal, 7Z-Pentadecenal, 8Z-Hexadecenal, 9Z-Hexadecenal, 10Z-Heptadecenal, 5Z,8Z-Tetradecadienal, 6Z,9Z-Pentadecadienal, 7*Z*,10*Z*-Tetradecadienal, 8Z,11Z-Pentadecadienal, 7Z,9E-Hexadecadienal, 8*Z*,10*E*-Heptadecadienal, 8*E*,10*Z*-Hexadecadienal, 9*E*,11*Z-*Heptadecadienal, 9-Methylundecanal, 10-Methylundecanal, 10-Methyldodecanal, 11-Methyldodecanal, 11-Methyltridecanal, 12-Methyltridecanal, 12-Methyltetradecanal, 13-Methyltetradecanal sowie 13-Methylpentadecanal sein.

Im Kontext der vorliegenden Erfindung umfassen alle Verbindungen, in denen die Positionen der Doppelbindung hinsichtlich der (Z)/(E)-Konfiguration nicht spezifisch angegeben ist, alle möglichen Positionen der Doppelbindung und Mischungen der relevanten Verbindungen.

Gemäß einer weiteren Ausführungsform des erfindungsgemäßen Verfahrens kann der mindestens eine rekombinante Mikroorganismus ausgewählt sein aus der Gruppe bestehend aus *Escherichia coli,* bevorzugt E. *coli* BL21, E. *coli* MG1655, E. *coli* W3110 und deren Abkömmlinge, *Bacillus* spp., bevorzugt B. *licheniformis, B. subitilis* oder B. *amyloliquefaciens*, und deren Abkömmlinge, *Saccharomyces* spp., bevorzugt S. *cerevesiae,* und deren Abkömmlinge, *Hansenula* bzw. *Komagatella* spp., bevorzugt K. *phaffii und H. polymorpha,* und deren Abkömmlingen, *Kluyveromyces* spp, bevorzugt K. *lactis.*

Gemäß einer bevorzugten Ausführungsform kann zusätzlich mindestens eine NADH-Oxidase und/oder mindestens eine Lipase bereitgestellt werden.

NADH-Oxidasen sind solche Enzyme, die aufgrund ihrer Substratspezifität und Regioselektivität die Oxidation eines Aldehyds katalysieren können. Diese können in einer bevorzugten Ausführungsform des erfindungsgemäßen Verfahrens als zusätzliche Enzyme eingesetzt werden, um die eng aufeinander abgestimmten Verfahren durch das Recycling des entsprechenden Co-Faktors noch effizienter zu gestalten.

In einer weiteren bevorzugten Ausführungsform des erfindungsgemäßen Verfahrens kann die mindestens eine NADH-Oxidase eine Aminosäuresequenz umfassen, wobei die Aminosäuresequenz unabhängig ausgewählt ist aus der Gruppe bestehend aus SEQ ID NOs: 5 und 6, oder einer Sequenz mit mindestens 90%, 91%, 92%, 93%, 94%, 95%, 96%, 97%, 98%, 99% oder 100% Sequenzidentität dazu. In einer Ausführungsform wird die die NADH Oxidase kodierende Aminosäuresequenz kodiert von einer Nukleinsäuresequenz, ausgewählt aus der Gruppe bestehend aus SEQ ID NOs: 7 und 8, oder einer Sequenz mit mindestens 90%, 91%, 92%, 93%, 94%, 95%, 96%, 97%, 98%, 99% oder 100% Sequenzidentität dazu.

Darüber hinaus kann ein Enzym, das nach den verschiedenen Aspekten und Ausführungsformen der vorliegenden Erfindung die erfindungsgemäße Reaktion katalysiert, eine katalytisch aktive Domäne oder ein Fragment des jeweiligen Enzyms sein, von dem es stammt.

In einer weiteren bevorzugten Ausführungsform kann die mindestens eine Lipase eine kommerzielle Lipase ausgewählt aus der Gruppe bestehend aus *Candida antarctica*, *Aspergillus niger, Rhizopus oryzae, Penicillium camembertii, Mucor juvanicus*, *Penicillium roqueforti*, Schweinepankreas, *Candida rugosa*, *Rhizomucor miehei*, *Candida antarctica* oder *Rhizopus delemar* sein.

In einer weiteren bevorzugten Ausführungsform kann die mindestens eine Aldehyddehydrogenase eine Aminosäuresequenz umfassen bzw. aus dieser bestehen, wobei die Aminosäuresequenz ausgewählt sein kann aus der Gruppe bestehend aus SEQ ID NOs: 9, 10, 11, 12, 13, 14 oder 15 oder einer Sequenz mit mindestens 90%, 91%, 92%, 93%, 94%, 95%, 96%, 97%, 98%, 99% oder 100% Sequenzidentität dazu.

Eine weitere Ausführungsform der vorliegenden Erfindung kann auf Nukleinsäuresequenzen oder Abschnitte davon gerichtet sein, die für Polypeptide mit enzymatischer Funktion kodieren, welche zum Zwecke der Reinigung, Sekretion, Detektion oder Lokalisation der mindestens einen alpha-Dioxygenase und/oder der mindestens einen Aldehyddehydrogenase und/oder der mindestens einen Lipase und/oder der mindestens einen NADH-Oxidase dienen. Diese Nukleinsäureabschnitte werden auch als tag-Sequenzen bezeichnet und können den Nukleinsäureabschnitten, die für die mindestens eine alpha-Dioxygenase und/oder die mindestens eine Aldehyddehydrogenase kodieren vorangestellt (N-terminal) und/oder nachgestellt (C-terminal) sein. Bevorzugt sind tag-Sequenzen ausgewählt aus der folgenden Liste: Polyhistidin(His)-Tag, Glutathion-S-transferase (GST)-tag, Thioredoxin-Tag, FLAG-tag, *green fluorescent protein* (GFP)-tag, Streptavidin-Tag, Maltose-Bindeprotein(MBP)-Tag, Chloroplastentransitpeptid, Mitochondrientransitpeptid und/oder Sekretionstag. Der Fachmann kennt überdies eine Reihe weiterer geeigneter tag-Sequenzen für Mikroorganismen oder Pilze von Interesse als Produktionsstamm, wobei diese tag-Sequenzen die Sekretion eines Polypeptids von Interesse unmittelbar in den Kulturüberstand erlauben. Dies mag in einigen Ausführungen vorteilhaft sein, um ein Polypeptid von Interesse leichter erhalten und optional reinigen zu können.

In einem weiteren Aspekt der vorliegenden Erfindung betrifft die Erfindung ein Vektorsystem, bevorzugt ein Plasmidvektorsystem, welches mindestens einen Vektor oder Plasmidvektor umfassend einen Nukleinsäureabschnitt (a) umfassend mindestens ein für eine alpha-Dioxygenase kodierendes Gen mit einer Nukleinsäuresequenz gemäß einer der SEQ ID NO: 3 oder 4 oder einer Nukleinsäuresequenz mit einer Ähnlichkeit von 90 %, 91 %, 92 %, 93 %, 94 %, 95 %, 96 %, 97%, 98 %, 99% oder mehr zu einer der SEQ ID NO: 3 oder 4 umfasst, sowie einen Nukleinsäureabschnitt (b) umfassend mindestens ein für eine Aldehyddehydrogenase kodierendes Gen; und optional als Teil des Nukleinsäureabschnitts (a) und/oder (b) einen Nukleinsäureabschnitt umfasst, welcher mindestens ein für eine NADH-Oxidase kodierendes Gen und/oder einen Nukleinsäureabschnitt umfassend mindestens ein für eine Lipase kodierendes Gen umfasst, wobei der Nukleinsäureabschnitt (a) und/oder der Nukleinsäureabschnitt (b) auf dem gleichen Vektor, oder auf zwei oder mehreren separaten Vektoren bereitgestellt wird.

In einem weiteren Aspekt betrifft die vorliegende Erfindung eine Zusammensetzung umfassend eine Aldehydmischung erhalten durch ein erfindungsgemäßes Verfahren, dadurch gekennzeichnet, dass die Zusammensetzung mindestens ein oder bevorzugt mindestens zwei oder drei Aldehyde, ausgewählt aus der Gruppe bestehend aus 7Z-Tetradecenal, 8Z-Pentadecenal, Pentadecanal, 7Z-Hexadecenal und 8Z-Heptadecenal umfasst, wobei der eine oder die mehreren Aldehyd(e) jeweils in einem Anteil von ungefähr 0,0001 - 20 Gew.-%, bevorzugt 0,001 - 10 Gew.-%, besonders bevorzugt 0,01 - 5 Gew.-%, jeweils in Bezug auf die Gesamtzusammensetzung, vorliegt und wobei die Zusammensetzung optional weitere Aldehyde erhalten durch ein Verfahren nach einem der Ansprüche 1 bis 12 enthält, oder wobei die Zusammensetzung 7Z-Tetradecenal in einem Anteil von ungefähr 0,0001 - 20 Gew.-% umfasst, oder wobei die Zusammensetzung 8Z-Pentadecenal in einem Anteil von ungefähr 0,0001 - 20 Gew.-% umfasst, in Bezug auf die Gesamtzusammensetzung, oder wobei die Zusammensetzung Pentadecanal in einem Anteil von ungefähr 0,0001 - 20 Gew.-% umfasst, in Bezug auf die Gesamtzusammensetzung, oder wobei die Zusammensetzung 7Z-Hexadecenal in einem Anteil von ungefähr 0,0001 - 20 Gew.-% umfasst, in Bezug auf die Gesamtzusammensetzung, oder wobei die Zusammensetzung 8Z-Heptadecenal in einem Anteil von ungefähr 0,0001 - 20 Gew.-% umfasst, in Bezug auf die Gesamtzusammensetzung, oder wobei die Zusammensetzung mindestens 8Z-Pentadecenal und Pentadecanal umfasst, wobei der Anteil an 8Z-Pentadecenal und Pentadecanal in der Gesamtzusammensetzung ungefähr 0,0001 - 20 Gew.-% umfasst, oder wobei die Zusammensetzung mindestens 8Z-Pentadecenal und 8Z-Heptadecenal umfasst, wobei der Anteil an 8Z-Pentadecenal und 8Z-Heptadecenal in der Gesamtzusammensetzung ungefähr 0,0001 - 20 Gew.-% umfasst.

Eine Zusammensetzung im Kontext der vorliegenden Erfindung ist eine Halbfertig- oder Fertigwarenzusammensetzung und ist bevorzugt ausgewählt aus der Gruppe bestehend aus der Ernährung oder dem Genuss dienende Zubereitung oder einer kosmetischen oder reinigenden Zubereitung.

Der Ernährung oder dem Genuss dienende Zubereitungen im Sinne der vorliegenden Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke, Instant-Fruchtgetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Buttermilchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett-und Ölbasis oder Emulsionen derselben (z.B. Mayonnaise, Remoulade, Dressings), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Eine kosmetische oder reinigende Zubereitung im Sinne der vorliegenden Erfindung ist eine Zubereitung, welche zur Reinigung, Pflege oder Parfümierung des Körpers oder zur Reinigung verwendet werden kann. Bevorzugte kosmetische Zubereitungen sind ausgewählt aus der Gruppe bestehend aus Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom ÖI-in-Wasser-, vom Wasser-in-ÖI- und vom Wasser-in-ÖI-in-Wasser-Typ wie z.B. Hautcremes- und -lotionen, Gesichtscremes und -lotionen, Sonnenschutzcremes-und - lotionen, After-sun-cremes und -lotionen, Handcremes und -lotionen, Fußcremes und - lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

In einer bevorzugten Ausführungsform enthält die erhaltene Zusammensetzung je nach Ausführungsform des erfindungsgemäßen Verfahrens weitere Aldehyde, Carbonsäuren oder Alkohole, ausgewählt aus der Gruppe bestehend aus Dodecanol, Tridecanal, Tridecanol, Tetradecanal, 8Z-Tetradecenol, Tetradecanol, Pentadecanal, Pentadecanol, Octansäure, Decansäure, Tridecanal, Tridecensäure, Pentadecenal und 8Z-Tetradecensäure.

In einer weiteren Ausführungsform können die Einzelkomponenten der erhaltenen Mischungen mit einem dem Fachmann bekannten Verfahren partiell oder vollständig gereinigt werden. Ein großer Vorteil dieser Mischungen ist demnach die universelle Einsetzbarkeit in einer Vielzahl von Industriezweigen, da diese sowohl als Mischung als auch als Einzelkomponenten verwendet werden können.

Nachfolgend wird die vorliegende Erfindung anhand von nicht-limitierenden Beispielen und basierend auf der im Sequenzprotokoll und den Abbildungen bereitgestellten Offenbarung weiter erläutert.

### Beispiele

### Beispiel 1: Expression der alpha-Dioxygenasen gemäß SEQ ID NO: 1 oder 2

Die Nukleotidsequenzen gemäß SEQ ID NO: 3 (LepDOX) und 4 (CaIDOX) wurden in den Expressionsvektor pET28a (Novagen, Darmstadt, Deutschland) kloniert. Die erhaltenen Vektoren wurden in kompetente E. *coli* BL21 (DE3)-Zellen transformiert. *E*. *coli*-Zellen, die nach Selektion die α-DOX-Gene enthielten, wurden in LB-Medien mit 50 µg/mL Kanamycin kultiviert. Die Induktion der Proteinexpression erfolgte durch Zugabe von 0,5 mM Isopropyl-β-D-1-thiogalactopyranosid (IPTG), woraufhin die Zellen 18 Stunden lang bei 18°C inkubiert und durch Zentrifugation bei 4.500 g und 4°C für 20 Minuten geerntet wurden.

Anschließend wurden die Zellpellets in Puffer (50 mM Tris-HCI, 200 mM NaCl und 20 mM Imidazol; pH 8,0) resuspendiert und mit einem Ultraschallgerät aufgeschlossen. Während der Zelllyse für die α-DOX wurde 1 % (v/v) Triton X-100 zugesetzt, um die Extraktion zu erleichtern. Das Gesamtzelllysat wurde zweimal bei 10.000 x g und 4°C für 30 Minuten zentrifugiert, und der Überstand, der den löslichen Rohextrakt enthielt, wurde auf eine Säule mit Ni-Agaroseharz (Carl Roth, Karlsruhe, Deutschland) aufgetragen und durch immobilisierte Metallionen-Affinitätschromatographie (IMAC) gereinigt. Die His-markierten Proteine wurden durch Zugabe von Puffer B (50 mM Tris-HCI, 200 mM NaCl und 300 mM Imidazol; pH 8,0) eluiert. Die Proteine wurden zur Überprüfung ihrer Expression und Reinheit mittels Natriumdodecylsulfat-Polyacrylamid-Gelelektrophorese (SDS-PAGE) analysiert. Die Proteinkonzentrationen wurden mit dem Bradford-Assay unter Verwendung von Rinderserumalbumin (BSA) als Standard (Carl Roth) quantifiziert.

### Beispiel 2: Substrat-Spektrum der alpha-Dioxyqenasen gemäß SEQ ID NO: 1 oder 2

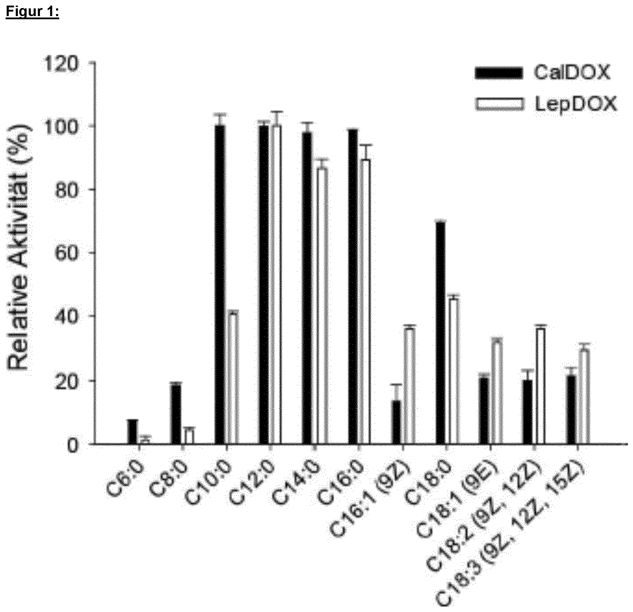

Die zwei α-DOX gemäß SEQ ID NO: 1 (LepDOX) oder SEQ ID NO: 2 (CaIDOX) wurden mit verschiedenen Fettsäuren mit einer Kettenlänge von 6 Kohlenstoffatomen bis 18 Kohlenstoffatomen inkubiert und zeigten eine maximale relative Aktivität für Laurinsäure (C12), Myristinsäure (C14) und Palmitinsäure (C16). Weitere gute Aktivitäten konnten für kurzkettige Fettsäuren (C8 bis C 10) festgestellt werden (Figur 1).

### Beispiel 3: Ganzzell-Biotransformation

Die Expression des rekombinanten CaIDOX- und LepDOX-Enzyms wurde in *E*. *coli*-Zellen wie in Beispiel 1 induziert. Nach der Ernte der Zellen durch Zentrifugation bei 9.600 x g und 4°C für 10 Minuten wurden die Zellen mit einem Medium gewaschen, das 200 mM Kaliumphosphat (pH 7,4) mit 50 mM NaCl enthielt, und anschließend unter denselben Bedingungen zentrifugiert. Nach dem Dekantieren des Überstandes wurden die Zellen in dem gleichen Medium resuspendiert.

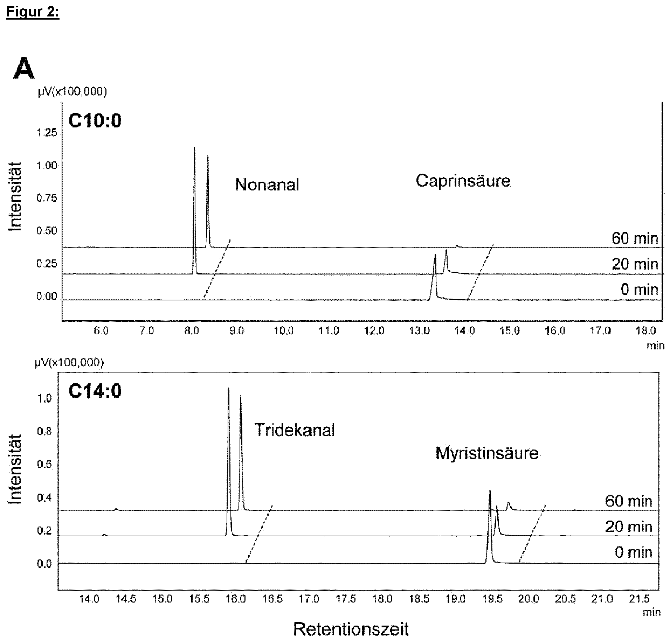

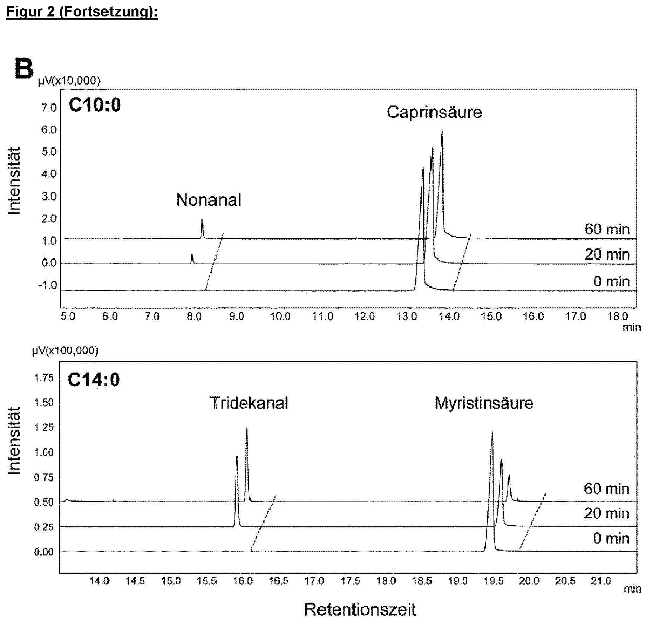

Ganzzell-Biotransformationen wurden mit *E*. *coli*-Zellen bei 12,5 g Nassgewicht in Puffer bei 35°C und 1.000 rpm mit einem Reaktionsvolumen von 300 µL unter Verwendung eines ThermoMixers (Eppendorf, Hamburg, Deutschland) durchgeführt. Die Reaktion wurde durch Zugabe von 15 µl 100 mM Fettsäuresubstraten (Caprinsäure oder Myristinsäure) aus einer DMSO-Stammlösung zu 285 µl der ruhenden Zellsuspension eingeleitet. Nach der Inkubation für die angegebenen Zeiten wurde die Biotransformation durch Zugabe von 30 µl 2 M HCl gestoppt, und die Proben wurden dreimal mit einem gleichen Volumen Ethylacetat extrahiert und über wasserfreiem Na2SO4 getrocknet und analysiert. Es konnte eine Umsetzung von beiden Fettsäuren innerhalb von 20 (C10) oder 40 (C14) Minuten beobachtet werden (Figur 2).

## Patentansprüche

1. Biotechnologisches Verfahren zur Herstellung mindestens eines ungesättigten Aldehyds und dessen korrespondierender Carbonsäure und/oder mindestens eines gesättigten Aldehyds und dessen korrespondierender Carbonsäure, wobei das Verfahren das Bereitstellen mindestens einer alpha-Dioxygenase mit einer Aminosäuresequenz gemäß SEQ ID NO: 1 oder 2 oder einer Aminosäuresequenz mit einer Ähnlichkeit von 90 %, 91 %, 92 %, 93 %, 94 %, 95 %, 96 %, 97%, 98 %, 99% oder mehr zu SEQ ID NO: 1 oder 2 und mindestens einer Aldehyddehydrogenase umfasst.

2. Verfahren nach Anspruch 1, wobei das biotechnologische Verfahren ein fermentatives Verfahren ist, umfassend die Schritte:
i. Bereitstellen mindestens eines rekombinanten Mikroorganismus oder Pilzes enthaltend einen Nukleinsäure-Abschnitt, umfassend mindestens ein für eine Aldehyddehydrogenase kodierendes Gen und/oder für eine alpha-Dioxygenase kodierendes Gen mit einer Nukleinsäuresequenz gemäß einer der SEQ ID NO: 3 oder 4 oder einer Nukleinsäuresequenz mit einer Ähnlichkeit von 90 %, 91 %, 92 %, 93 %, 94 %, 95 %, 96 %, 97%, 98 %, 99% oder mehr zu einer der SEQ ID NO: 3 oder 4;
ii. Kultivieren des mindestens einen rekombinanten Mikroorganismus unter Bedingungen, die die Expression des entsprechenden Expressionsproduktes erlauben;
iii. Hinzufügen mindestens einer Carbonsäure oder mindestens einer mit einem Alkohol veresterten Carbonsäure als Edukt zu dem mindestens einen kultivierten, rekombinanten Mikroorganismus;
iv. Erhalten mindestens eines ungesättigten Aldehyds und dessen korrespondierender Carbonsäure und/oder mindestens eines gesättigten Aldehyds und dessen korrespondierender Carbonsäure durch Reaktion der mindestens einen alpha-Dioxygenase und der mindestens einen Aldehyddehydrogenase mit der mindestens einen Carbonsäure oder der mit einem Alkohol veresterten Carbonsäure aus Schritt iii.

3. Verfahren nach Anspruch 1, wobei das biotechnologische Herstellungsverfahren ein enzymatisches Verfahren ist, umfassend die Schritte:
i. Bereitstellen mindestens einer alpha-Dioxygenase mit einer Aminosäuresequenz gemäß SEQ ID NO: 1 oder 2 oder einer Aminosäuresequenz mit einer Ähnlichkeit von 90 %, 91 %, 92 %, 93 %, 94 %, 95 %, 96 %, 97%, 98 %, 99% oder mehr zu einer der SEQ ID NO: 1 oder 2 und mindestens einer Aldehyddehydrogenase, wobei die mindestens eine alpha-Dioxygenase und/oder die mindestens eine Aldehyddehydrogenase natürlich, chemisch oder biotechnologisch hergestellt sein kann/können;
ii. Hinzufügen mindestens einer Carbonsäure oder mindestens einer mit einem Alkohol veresterten Carbonsäure zu den bereitgestellten Enzymen aus Schritt i;
iii. Erhalten mindestens eines ungesättigten Aldehyds und dessen korrespondierender Carbonsäure und/oder mindestens eines gesättigten Aldehyds und dessen korrespondierender Carbonsäure durch Reaktion der mindestens einen alpha-Dioxygenase und der mindestens einen Aldehyddehydrogenase mit der mindestens einen Carbonsäure oder der mit einem Alkohol veresterten Carbonsäure aus Schritt ii.

4. Verfahren nach einem der vorangehenden Ansprüche, wobei mindestens eines der Edukte biotechnologischen, natürlichen oder chemischen Ursprungs ist, oder eine Kombination davon ist.

5. Verfahren nach einem der vorangehenden Ansprüche, wobei die Edukte ausgewählt sind aus der Gruppe bestehend aus vollständig gesättigten, ein-, zwei- oder dreifach ungesättigten oder einfach verzweigten Fettsäuren von C6 bis C20, bevorzugt von C8 bis C18, besonders bevorzugt aus der Gruppe bestehend aus Caprylsäure, Caprinsäure, 4-Ethyloctansäure, Laurinsäure, 10-Methylundecansäure, 9-Methylundecansäure, 10-Methyldodecansäure, 11-Methyldodecansäure, 11-Methyltridecansäure, 12-Methyltridecansäure, 13-Methyltetradecansäure, 12-Methyltetradecansäure, 13-Methylpentadecansäure, 14-Methylpentadecansäure, 14-Methylhexadecansäure, 15-Methylhexadecansäure, Myristinsäure, Palmitinsäure, Stearinsäure, Palmitoleinsäure, Petroselinsäure, Margarinsäure, cis-Vaccensäure, Linolsäure, Stearidonsäure, gamma-Linolensäure, alpha-Linolensäure, Ölsäure, Punicinsäure, alpha-Elaeostearinsäure sowie der zugrundeliegenden fetten Fettsäureester, insbesondere die fetten Öle ausgewählt aus Olivenöl, Erucasäure-armem Rapsöl, Macadamianussöl, Sanddornfruchtfleischöl, Borretschöl, Johannisbeerkernöl, Petersiliensamenöl, Dillsamenöl, Granatapfelkernöl, Kokosnussöl, Sonnenblumenöl, Weizenkeimöl, Reiskeimöl, Erdnussöl, Sesamöl, Palmfruchtöl, Traubenkernöl sowie Pilzöle, gewonnen aus einer beliebigen der Gattungen, ausgewählt aus *Conidiobolus, Flammulina, Fomes, Ganoderma, Mortierella, Panellus, Pleurotus, Psathyrella, Stereum, Umbelopsis* und deren Derivate der Carbonsäuren und der mit Alkoholen veresterten Carbonsäuren, bevorzugt, wobei die Edukte ausgewählt sind aus der Gruppe bestehend aus Palmitoleinsäure, Arachidonsäure, alpha-Linolensäure, Ölsäure, Punicinsäure, alpha-Elaeosterarinsäure, Docosahexaensäure, Eicosapentaensäure, Petroselinsäure, Chaulmoograsäure, alpha-Licarsäure, Olivenöl, Rapsöl, Macadamianussöl, Sanddornfruchtfleischöl, Tungöl, Fischöl, Borretschöl, Chaulmoograöl, Petersilienöl, Oiticicaöl, Granatapfelkernöl, Kokosnussöl, Sonnenblumenöl, Traubenkernöl sowie Pilzöle, gewonnen aus einer beliebigen der Gattungen, ausgewählt aus *Conidiobolus, Flammulina, Fomes, Ganoderma, Mortierella, Panellus, Pleurotus, Psathyrella, Stereum, Umbelopsis* und deren Derivate sind.

6. Verfahren nach einem der vorangehenden Ansprüche, wobei das Produkt des Verfahrens mindestens ein gesättigter Aldehyd und dessen korrespondierende Carbonsäure und/oder mindestens ein ungesättigter Aldehyd und dessen korrespondierende Carbonsäure ist, ausgewählt aus der Gruppe bestehend aus 7Z,10Z-Hexadecadienal, 8Z,11Z-Heptadecadienal, 6*E*,9*E*)-Pentadecadienal, 7Z-Hexadecenal, 8Z-Pentadecenal, 8Z,11Z,14Z-Heptadecatrienal, 7Z,10Z,13Z-Hexadecatrienal, 4Z,7Z,10Z,13Z-Nonadecatetraenal, 8*Z*,10*E*,12*E-*Heptadecatrienal, 8*Z*,10*E*,12*Z*-Heptadecatrienal, 7*Z*,9*E*,11*Z*-Hexadecatrienal, 7*Z*,9*E*,11*E*-Hexadecatrienal, 9-Decenal, 10-Undecenal, 3Z-Decenal, 4Z-Undecenal, 7Z-Dodecenal, 8Z-Tridecenal, 7Z-Tetradecenal, 8Z-Pentadecenal, 9Z-Tetradecenal, 10Z-Pentadecenal, 7Z-Pentadecenal, 8Z-Hexadecenal, 9Z-Hexadecenal, 10*Z*-Heptadecenal, 5Z,8Z-Tetradecadienal, 6Z,9Z)-Pentadecadienal, 7*Z*,10*Z*-Tetradecadienal, 8Z,11Z-Pentadecadienal, 7Z,9E-Hexadecadienal, 8*Z*,10*E*-Heptadecadienal, 8*E*,10*Z*-Hexadecadienal, 9E,11Z-Heptadecadienal, 9-Methylundecanal, 10-Methylundecanal, 10-Methyldodecanal, 11-Methyldodecanal, 11-Methyltridecanal, 12-Methyltridecanal, 12-Methyltetradecanal, 13-Methyltetradecanal sowie 13-Methylpentadecanal.

7. Verfahren nach einem der Ansprüche 2 bis 6, umfassend die weiteren Schritte:
(a) Erhalten mindestens einer Carbonsäure, bevorzugt mindestens einer Carbonsäure wie definiert in Anspruch 6, bevorzugt umfassend eine ungerade Anzahl an Kohlenstoffatomen, als Produkt;
(b) optional: Reinigen der mindestens einen als Produkt erhaltenen Carbonsäure;
(c) Verwenden der mindestens einen als Produkt erhaltenen Carbonsäure als modifiziertes Edukt zur Durchführung eines biotechnologischen Verfahrens zur Herstellung mindestens eines ungesättigten Aldehyds und dessen korrespondierender Carbonsäure und/oder mindestens eines gesättigten Aldehyds und dessen korrespondierender Carbonsäure, wobei das Verfahren das Bereitstellen mindestens einer alpha-Dioxygenase und mindestens einer Aldehyddehydrogenase umfasst.

8. Verfahren nach einem der vorangehenden Ansprüche, wobei der mindestens eine rekombinante Mikroorganismus ausgewählt ist aus der Gruppe bestehend aus *Escherichia coli,* bevorzugt E. *coli* BL21, *E. coli* MG1655, E. *coli* W3110 und deren Abkömmlinge, *Bacillus* spp., bevorzugt *B. licheniformis, B. subitilis* oder *B. amyloliquefaciens*, und deren Abkömmlinge, *Saccharomyces* spp., bevorzugt S. *cerevesiae,* und deren Abkömmlinge, *Hansenula* bzw. *Komagatella* spp., bevorzugt K. *phaffii* und *H. polymorpha,* und deren Abkömmlingen, *Kluyveromyces* spp, bevorzugt K. *lactis.*

9. Verfahren nach einem der vorangehenden Ansprüche, wobei zusätzlich mindestens eine NADH-Oxidase und/oder mindestens eine Lipase bereitgestellt wird.

10. Verfahren nach Anspruch 8, wobei die mindestens eine NADH-Oxidase eine Aminosäuresequenz umfasst, wobei die Aminosäuresequenz ausgewählt ist aus der Gruppe bestehend aus SEQ IDs NOs: 5 und 6, oder einer Sequenz mit mindestens 90%, 91%, 92%, 93%, 94%, 95%, 96%, 97%, 98%, 99% oder 100% Sequenzidentität dazu.

11. Verfahren nach Anspruch 8, wobei die mindestens eine Lipase eine kommerzielle Lipase ausgewählt aus der Gruppe bestehend aus *Candida antarctica, Aspergillus niger, Rhizopus oryzae, Penicillium camembertii, Mucor juvanicus, Penicillium roqueforti*, Schweinepankreas, *Candida rugosa*, *Rhizomucor miehei*, *Candida antarctica* oder *Rhizopus delemar* ist.

12. Verfahren nach einem der vorangehenden Ansprüche, wobei die mindestens eine Aldehyddehydrogenase eine Aminosäuresequenz umfasst, wobei die Aminosäuresequenz ausgewählt ist aus der Gruppe bestehend aus SEQ ID NOs: 9, 10, 11, 12, 13, 14 oder 15 oder einer Sequenz mit mindestens 90%, 91%, 92%, 93%, 94%, 95%, 96%, 97%, 98%, 99% oder 100% Sequenzidentität dazu.

13. Vektorsystem, bevorzugt ein Plasmidvektorsystem, bestehend aus mindestens einem Vektor oder Plasmidvektor, umfassend einen Nukleinsäureabschnitt (a) umfassend mindestens ein für eine alpha-Dioxygenase kodierendes Gen mit einer Nukleinsäuresequenz gemäß einer der SEQ ID NO: 3 oder 4 oder einer Nukleinsäuresequenz mit einer Ähnlichkeit von 90 %, 91 %, 92 %, 93 %, 94 %, 95 %, 96 %, 97%, 98 %, 99% oder mehr zu einer der SEQ ID NO: 1 oder 2, sowie einen Nukleinsäureabschnitt (b) umfassend mindestens ein für eine Aldehyddehydrogenase kodierendes Gen; und optional umfassend als Teil des Nukleinsäureabschnitts (a) und/oder (b) einen Nukleinsäureabschnitt, umfassend mindestens ein für eine NADH-Oxidase kodierendes Gen und/oder einen Nukleinsäureabschnitt umfassend mindestens ein für eine Lipase kodierendes Gen, wobei der Nukleinsäureabschnitt (a) und/oder der Nukleinsäureabschnitt (b) auf dem gleichen Vektor, oder auf zwei oder mehreren separaten Vektoren bereitgestellt wird.
